# EUROPEAN PATENT SPECIFICATION

(11) **EP 1 738 656 B1**
(45) Date of publication and mention of the grant of the patent: **17.03.2010**
(21) Application number: 06115169.2
(22) Date of filing: 08.06.2006
(51) Int. Cl.: A23L 1/0522, A23L 1/0534, A23L 1/056, A23L 1/0528, A23L 1/00

(54) **Quick water-dissolving edible anti-hunger film containing fibres that swell in the presence of water**
Schnell wasserlöslicher Hunger unterdrückender essbarer Film, der in Wasser quellende Rohfasern enthält
Coupe-faim en forme de film comestible à dissolution rapide contenant des fibres qui gonflent en présence d'eau

(30) Priority: 29.06.2005 IT MI20051228
(43) Date of publication of application: 03.01.2007
(73) Proprietor: BIOFARMITALIA S.p.A., 20144 Milano (IT)
(72) Inventor: Pinna, Fausto, 20050, Lesmo (MI) (IT); Pinna, Marco, 21051, Arcisate (VA) (IT)
(74) Representative: Frignoli, Luigi

(56) References cited:
- EP-A1- 0 273 069
- EP-A1- 1 417 895
- US-A1- 2002 019 447
- US-A1- 2003 224 090
- US-A1- 2004 096 569
- US-A1- 2004 180 077
- US-A1- 2004 247 647
- DATABASE MEDLINE [Online] US NATIONAL LIBRARY OF MEDICINE (NLM), BETHESDA, MD, US; 2005, NI MHURCHU C ET AL: "Chitosan for overweight or obesity." XP002402857 Database accession no. NLM16034912 & COCHRANE DATABASE OF SYSTEMATIC REVIEWS (ONLINE) 2005, no. 3, 2005, page CD003892, ISSN: 1469-493X
- DATABASE MEDLINE [Online] US NATIONAL LIBRARY OF MEDICINE (NLM), BETHESDA, MD, US; November 2005 (2005-11), KEITHLEY JOYCE ET AL: "Glucomannan and obesity: a critical review." XP002402856 Database accession no. NLM16320857 & ALTERNATIVE THERAPIES IN HEALTH AND MEDICINE. 2005 NOV-DEC, vol. 11, no. 6, November 2005 (2005-11), pages 30-34, ISSN: 1078-6791
- XU Y X ET AL: "Chitosan-starch composite film: preparation and characterization" INDUSTRIAL CROPS AND PRODUCTS, ELSEVIER, NL, vol. 21, no. 2, March 2005 (2005-03), pages 185-192, XP004758840 ISSN: 0926-6690

## Description

The present invention relates to edible films partially quick-dissolving in water and on contact with human saliva, and containing natural fibres that swell in the presence of water, to give a satiating sense to the film user.

EP-A-1417895 in the name of the present applicants describes a film quick-dissolving in water and on contact with the human saliva, this film comprising at least one starch of high amylopectin content, at least one cellulose compatible with the starch and at least one cosmetic, aromatic or pharmaceutical substance or a substance with nutritive characteristics which are released within a very short time (a few seconds, generally from 2 to 15 seconds) after the film has been brought into contact with water.

US 2004/0096569 discloses edible thin films. Suitable water-soluble film formers include non-starch polysaccharides such as cellulose derivatives, chitosan but also native and modified starches.

The present invention provides a film which from certain aspects is similar (partially soluble within a very short time on contact with water or saliva) to that of EP-A-1417895, but differs considerably from it in that it comprises at least one natural food fibre which is insoluble in water and has the ability to swell to form semisolid particles (of volume between 0.01 and 1 cm³) on contact with water or with saliva, and still further on contact with the gastric juices of the stomach.

This film is substantially anhydrous and can usefully be made in the form of thin sheets having a thickness from 30 to 150 micron, a width from 1.5 to 3.5 cm and a length from 2 to 4 cm.

The present invention hence relates to an edible anti-hunger film partially quick-dissolving in water and containing fibres which swell in the presence of water, comprising between 4% and 40% of at least one starch of high amylopectin content, between 5% and 50% of at least one cellulose chosen from the group consisting of hydroxypropylmethylcellulose, hydroxyethylcellulose, hydroxypropylcellulose and carboxymethylcellulose, characterised by comprising between 1% and 60% of at least a natural food fibre swellable but insoluble in water and chosen from the group formed from high molecular weight chitosan and high molecular weight glucomannan, the percentages being by weight on a dry basis.

Preferably, the film also contains between 1% and 20% by weight on a dry basis of at least one soluble fibre chosen from pectins, gums, mucilages, hemicellulose, chitosan oligosaccharide and inulin.

Chitosan is a natural fibre derived from chitin, extracted from the shell of certain crustaceans: it attracts the fat molecules and sugar within the digestive tract, facilitating their elimination without any absorption into the body.

Glucomannan is a vegetable fibre, obtained from the root of the Konojak, which has the property of increasing the effectiveness of the chitosan. Moreover if taken with water it swells within the intestine to provide a satiating sense and facilitates its evacuation.

Inulin is a polysaccharide with the known property of facilitating digestion and reducing intestinal gas, by increasing the density of bifidobacteria and decreasing that of harmful bacteria.

The other soluble fibres (pectins, etc.), the presence of which as stated above is preferable, have a gelling power, which increases the viscosity and stickiness of the stomach contents, to delay gastric evacuation and slow down lipid and carbohydrate absorption, this being very useful in feeding individuals with altered lipid and glucide metabolism; these fibres also facilitate attainment of the satiety sensation within the framework of overall controlled diets aimed at controlling and reducing a person's weight.

The production process for the film of the invention is characterised by feeding water and at least one starch into a mixer, stirred at a temperature between 40°C and 50°C until the starch has dissolved and caramelized, adding at least one cellulose compatible with the starch and stirring to obtain a homogeneous mass, then cooling to a temperature between 25°C and 35°C, then finally adding firstly the insoluble fibre, then the possible inulin, then the soluble fibre and finally the flavouring until a viscosity of 10/25000 m.Pas is obtained, stirring to obtain a homogeneous mass which is spread with a doctor blade onto the surface of a support tape, heating in a through oven ventilated to a temperature between 30°C and 90°C to evaporate the residual water, cooling to ambient temperature, detaching the film from the support and punching it to the desired size (for example in the form of a 3x2 cm sheet, of 40-80 micron thickness).

The invention will be more apparent on reading the following examples, given by way of non-limiting example:

### EXAMPLE 1

The following formula is used:

| | % WET BASIS | WEIGHT (g) | % DRY BASIS |
|---|---|---|---|
| WATER | | | |
| HYDROXYPROPYLMETHYLCELLUL OSE | 14.4 | 111.7 | 26.64 |
| GLYCERIN | 4.6 | 35.7 | 8.51 |
| GUAR FLOUR | 3.5 | 27.2 | 6.48 |
| CHITOSAN OLIGOSACCHARIDE | 2.8 | 21.7 | 5.18 |
| OXIDIZED CORN STARCH | 2.8 | 21.7 | 5.18 |
| POLYSORBATE 80 | 2.8 | 21.7 | 5.18 |
| INULIN | 1.5 | 11.6 | 2.78 |
| HEAVY CHITOSAN | 1 | 7.8 | 1.85 |
| LIQUORICE FLAVOURING | 2.1 | 16.3 | 3.89 |
| ASPARTAME | 0.2 | 1.6 | 0.37 |

The amount of water in the mixture is such that its percentage by weight brings to 100% the sum of the percentage values of all the components of the mixture.

From the above Table it can be seen that the total weight (on dry basis) of all the fibres insoluble in water is of 1.86 %, that the total weight of the soluble fibres is of 7.94%, and that the cellulose derivative is present in an amount of 26.64%.

The components of two separate phases known as "Phase A" e "Phase B" are used
Phase A comprises:
   H₂O
   hydroxypropylmethylcellulose
   glycerin
   polysorbate 80
Phase B comprises:
   Guar flour
   heavy chitosan
   chitosan oligosaccharide
   Inulin

### Liquorice flavouring

The components of phase A are introduced into a closed jacketed mixer in the following succession: water, hydroxypropylmethylcellulose, oxidized starch, glycerin and polysorbate 80; these are stirred at medium speed while heating to 40°C, stirring being continued until a homogeneous solution is obtained.

While stirring, the temperature is brought to 35°C and stirring continued (at about 60 r.p.m.) for 30 minutes, heavy chitosan is added and stirring continued until the solution is homogeneous.

Chitosan polysaccharide, Guar flour, inulin and aspartame are added, maintaining stirring for 15 minutes. The temperature is adjusted to 30°C and the liquorice flavouring, previously mixed at ambient temperature, is slowly added. The mixture is stirred for 15 minutes. The mixed product is withdrawn by a peristaltic pump to enable it to flow onto a doctor blade heated to 30°C, through which there is passed a support tape of siliconized polyester, onto which the product is filmed to a thickness of 70 micron. The product (filmed on the polyester) is passed through an oven heated to 70°C and ventilated. At the oven exit the film is detached from the polyester support, punched with a roller punch into 2.2 x 3.2 cm rectangles, and the rectangles obtained inserted into a container which is sealed.

Each rectangle obtained has a thickness of 60 micron, with a dissolution time in the mouth of 6 seconds, while the heavy chitosan swells in the mouth (in contact with the saliva) after a further 3 seconds to form insoluble semisolid particles with a total volume of 0.05 cm³. These chitosan particles swell further within the stomach in the presence of gastric juices, to contribute to a sense of satiety for the film user.

### EXAMPLE 2

The following formula is used:

| | % WET BASIS | WEIGHT (g) | % DRY BASIS |
|---|---|---|---|
| WATER | | | |
| HYDROXYETHYLCELLULOSE | 3 | 23.3 | 5.55 |
| POLYVINIL PYRROLIDONE | 3 | 23.3 | 5.55 |
| GLYCERIN | 2 | 15.5 | 3.70 |
| GUAR FLOUR | 3.5 | 27.2 | 6.48 |
| CARRAGEENAN | 1 | 7.8 | 1.85 |
| OXIDISED CORN STARCH | 3 | 23.3 | 5.55 |
| POLYSORBATE 80 | 2.8 | 21.7 | 5.18 |
| INULIN | 1.4 | 10.9 | 2.59 |
| HIGH MOLECULAR WEIGHT GLUCOMANNAN | 32 | 248.3 | 59.21 |
| LIQUORICE FLAVOURING | 2.1 | 16.3 | 3.89 |
| ASPARTAME | 0.2 | 1.6 | 0.37 |

The amount of water in the mixture is such that its percentage by weight brings to 100% the sum of the percentage values of all the components of the mixture.

From the above Table it can be seen that the total weight (on dry basis) of all the fibres insoluble in water is of 59.23%, that the total weight of the soluble fibres is of 2.60%, and that the cellulose derivative is present in an amount of 5.56%.

The components of two separate phases known as "Phase A" and "Phase B" are used
Phase A comprises:
   H₂O
   Hydroxyethylcellulose
   Polyvinil pyrrolidone
   glycerin
   polysorbate 80
Phase B comprises:
   carrageenan
   high molecular weight glucomannan
   Guar flour
   Inulin
   green apple flavouring

The components of phase A are introduced into a closed jacketed mixer in the following succession: water, hydroxyethylcellulose, oxidized starch, polyvinyl pyrrolidone, glycerin and polysorbate 80 are first introduced; these are stirred at medium speed while heating to 40°C, stirring being continued until a homogeneous solution is obtained.

While stirring, the temperature is brought to 35°C and stirring continued (at about 60 r.p.m.) for 30 minutes, glucomannan is added and stirring continued at high speed (about 120 r.p.m.) until the solution is homogeneous. Colorant, carrageenan, Guar flour, inulin and aspartame are added, maintaining stirring for 15 minutes. The temperature is adjusted to 30°C and the green apple flavouring, previously mixed at ambient temperature, is slowly added. The mixture is stirred for 15 minutes. The mixed product is withdrawn by a peristaltic pump, to enable it to flow onto a doctor blade heated to 30°C, through which there is passed a support tape of siliconized polyester, onto which the product is filmed to a thickness of 60 micron. The product (filmed on the polyester) is passed through an oven heated to 70°C and ventilated. At the oven exit the film is detached from the polyester support, punched with a roller punch into 2.2 x 3.2 cm rectangles, and the rectangles obtained inserted into a container which is sealed.

Each rectangle obtained has a thickness of 60 micron, with a dissolution time in the mouth of 7 seconds, the formation of swollen glucomannan taking place after a further 6 seconds in the mouth, as soft expanded particles with a volume of about 1 cm³.

### EXAMPLE 3

| | % WET BASIS | WEIGHT (g) | % DRY BASIS |
|---|---|---|---|
| WATER | | | |
| HYDROXYPROPYLMETHYLCELLUL OSE | 13 | 100.9 | 24.05 |
| GLYCERIN | 5 | 38.8 | 9.25 |
| CARRAGEENAN | 6 | 46.6 | 11.10 |
| SOYA PECTIN | 3.5 | 27.2 | 6.48 |
| OXIDISED CORN STARCH | 3.5 | 27.2 | 6.48 |
| POLYETHYLENE GLYCOL 400 av | 2.8 | 21.7 | 5.18 |
| INULIN | 1.5 | 11.6 | 2.78 |
| HIGH MOLECULAR WEIGHT CHITOSAN | 1 | 7.8 | 1.85 |
| BANANA FLAVOURING | 2.5 | 19.4 | 4.63 |
| ASPARTAME | 0.2 | 1.6 | 0.37 |

The amount of water in the mixture is such that its percentage by weight brings to 100% the sum of the percentage values of all the components of the mixture.

From the above Table it can be seen that the total weight (on dry basis) of all the fibres insoluble in water is of 1.86%, that the total weight of the soluble fibres is of 9.24%, and that the cellulose derivative is present in an amount of 24.04%.

The components of two separate phases known as "Phase A" e "Phase B" are used
Phase A comprises:
   H₂O
   hydroxypropylmethylcellulose
   oxidised starch
   polyethylene glycol 400 av
   Soya pectin
Phase B comprises:
   high molecular weight chitosan
   carrageenan
   Soya pectin
   Inulin
   Banana flavouring

The components of phase A are introduced into a closed jacketed mixer in the following succession: water, oxidized starch, hydroxypropylmethylcellulose, polyethylene glycol 400 av; these are stirred at medium speed while heating to 40°C, stirring being continued until a homogeneous solution is obtained.

While stirring, the temperature is brought to 35°C and stirring continued (at about 60 r.p.m.) for 30 minutes, heavy chitosan is added and stirring continued at high speed (120 r.p.m.) until the solution is homogeneous.

Carrageenan, soya pectin and inulin are added, maintaining stirring for 15 minutes. The temperature is adjusted to 30°C and the banana flavouring, previously mixed at ambient temperature, is slowly added. The mixture is stirred for 15 minutes. The mixed product is withdrawn by a peristaltic pump to enable it to flow onto a doctor blade heated to 30°C, through which there is passed a support tape of siliconized polyester, onto which the product is filmed to a thickness of 60 micron. The product (filmed on the polyester) is passed through an oven heated to 70°C and ventilated. At the oven exit the film is detached from the polyester support, punched with a roller punch into 2.2 x 3.2 cm rectangles, and the rectangles obtained inserted into a container which is sealed.

Each rectangle obtained has a thickness of 60 micron, with a dissolution time in the mouth of 6 seconds, formation of swollen chitosan particles taking place after a further 7 seconds (the particles have a volume of about 0.06 cm³).

### EXAMPLE 4

The following formula is used:

| | % WET BASIS | WEIGHT (g) | % DRY BASIS |
|---|---|---|---|
| WATER | | | |
| HYDROXYPROPYLMETHYLCELLUL OSE | 12.96 | 111.7 | 27.73 |
| GLYCERIN | 4.14 | 35.7 | 8.86 |
| GUAR FLOUR | 3.16 | 27.2 | 6.75 |
| CHITOSAN OLIGOSACCHARIDE | 3.02 | 26 | 6.45 |
| OXIDISED CORN STARCH | 2.52 | 21.7 | 5.39 |
| POLYSORBATE 80 | 2.52 | 21.7 | 5.39 |
| INULIN | 1.86 | 16 | 3.97 |
| HEAVY CHITOSAN | 9.86 | 85 | 21.10 |
| LIQUORICE FLAVOURING | 1.89 | 16.3 | 4.05 |
| ASPARTAME | 0.19 | 1.6 | 0.40 |

The amount of water in the mixture is such that its percentage by weight brings to 100% the sum of the percentage values of all the components of the mixture.

From the above Table it can be seen that the total weight (on dry basis) of all the fibres insoluble in water is of 21.10%, that the total weight of the soluble fibres is of 10.43%, and that the cellulose derivative is present in an amount of 27.73%.

The some procedure described in Example 1 above is followed, while using the components and their amounts specified in this Example. Substantially the same results and an edible anti-hunger fibre as detailedly indicated in Example 1 are obtained.

### EXAMPLE 5

The following formula is used:

| | % WET BASIS | WEIGHT (g) | % DRY BASIS |
|---|---|---|---|
| WATER | | | |
| HYDROXYETHYLCELLULOSE | 3.68 | 23.3 | 7.63 |
| POLYVINIL PYRROLIDONE | 3.68 | 23.3 | 7.63 |
| GLYCERIN | 2.44 | 15.5 | 5.07 |
| GUAR FLOUR | 4.29 | 27.2 | 8.90 |
| CARRAGEENAN | 1.23 | 7.8 | 2.55 |
| OXIDISED CORN STARCH | 3.68 | 23.3 | 7.63 |
| POLYSORBATE 80 | 3.42 | 21.7 | 7.10 |
| INULIN | 6.62 | 42 | 13.75 |
| HIGH MOLECULAR WEIGHT GLUCOMANNAN | 11.83 | 75 | 24.55 |
| LIQUORICE FLAVOURING | 2.57 | 16.3 | 5.33 |
| ASPARTAME | 0.25 | 1.6 | 0.52 |

The amount of water in the mixture is such that its percentage by weight brings to 100% the sum of the percentage values of all the components of the mixture.

From the above Table it can be seen that the total weight (on dry basis) of all the fibres insoluble in water is of 24.55%, that the total weight of the soluble fibres is of 13.75%, and that the cellulose derivative is present in an amount of 7.63%.

The same procedure described in Example 2 above is followed while using the components and their amounts as specified in this Example: an edible anti-hunger film having substantially the same features mentioned in Example 2 is obtained.

## Claims

1. An edible anti-hunger film partially quick-dissolving in water and containing fibres which swell in the presence of water, comprising between 4% and 40% of at least one starch of high amylopectin content, between 5% and 50% of at least one cellulose chosen from the group consisting of hydroxypropylmethylcellulose, hydroxyethylcellulose, hydroxypropylcellulose and carboxymethylcellulose, **characterised by** comprising between 1% and 60% of at least one natural food fibre swellable but insoluble in water and chosen from the group formed from high molecular weight chitosan and high molecular weight glucomannan, the percentages being by weight on a dry basis.

2. An edible film as claimed in claim 1, **characterised by** containing between 1% and 20% by weight on a dry basis of at least one soluble fibre chosen from pectins, gums, mucilages, hemicellulose, chitosan oligosaccharide and inulin.

3. An edible film as claimed in claims 1 and 2, **characterized by** containing between 20% and 25% of said natural food fibre swellable but insoluble in water.

4. An edible film as claimed in claims 2 and 3, **characterized by** containing between 10% and 14% of said soluble fibre.

## Patentansprüche

1. Hunger unterdrückender essbarer Film, der partiell schnell wasserlöslich ist und Fasern enthält, die in Gegenwart von Wasser quellen, umfassend zwischen 4% und 40% wenigstens einer Stärke mit hohem Amylopektingehalt, zwischen 5% und 50% wenigstens einer Cellulose, ausgewählt aus der Gruppe, bestehend aus Hydroxypropylmethylcellulose, Hydroxyethylcellulose, Hydroxypropylcellulose und Carboxymethylcellulose, **dadurch gekennzeichnet, dass** er zwischen 1% und 60% wenigstens einer natürlichen Nahrungsmittelfaser umfasst, die in Wasser quellbar, aber unlöslich ist und ausgewählt ist aus der Gruppe, bestehend aus Chitosan mit hohem Molekulargewicht und Glucomannan mit hohem Molekulargewicht, wobei die Prozentangaben Gewichtsprozent, bezogen auf die Trockensubstanz, sind.

2. Essbarer Film, wie er in Anspruch 1 beansprucht ist, **dadurch gekennzeichnet, dass** er zwischen 1 Gew.-% und 20 Ges.-%, bezogen auf die Trockensubstanz, wenigstens einer löslichen Faser enthält, die ausgewählt ist aus Pektinen, Gummen, Pflanzenschleimen, Hemicellulose, Chitosanoligosaccharid und Inulin.

3. Essbarer Film, wie er in den Ansprüchen 1 und 2 beansprucht ist, **dadurch gekennzeichnet, dass** er zwischen 20% und 25% der natürlichen Nahrungsmittelfaser enthält, die in Wasser quellbar, aber unlöslich ist.

4. Essbarer Film, wie er in den Ansprüchen 2 und 3 beansprucht ist, **dadurch gekennzeichnet, dass** er zwischen 10% und 14% der löslichen Faser enthält.

## Revendications

1. Film coupe-faim comestible à dissolution rapide partielle dans l'eau et contenant des fibres qui gonflent en présence d'eau, comprenant entre 4% et 40% d'au moins un amidon à haute teneur en amylopectine, entre 5% et 50% d'au moins une cellulose choisie parmi le groupe composé des hydroxypropylméthylcellulose, hydroxyéthylcellulose, hydroxypropylcellulose et carboxyméthylcellulose, **caractérisé par le fait qu'**il comprenne entre 1% et 60% d'au moins une fibre alimentaire capable de gonfler mais insoluble dans l'eau et choisie parmi le groupe formé à partir des chitosan haute densité et glucomannan haute densité, les pourcentages étant en poids sur une base sèche.

2. Film comestible selon la revendication 1, **caractérisé par le fait qu'**il contienne entre 1% et 20% en poids sur une base sèche d'au moins une fibre soluble choisie parmi des pectines, des gommes, des mucilages, de l'hémicellulose, du chitosan, de l'oligosaccharide et de l'inuline.

3. Film comestible selon les revendications 1 et 2, **caractérisé par le fait qu'**il comprenne entre 20% et 25% de ladite fibre alimentaire capable de gonfler mais insoluble dans l'eau.

4. Film comestible selon les revendications 2 et 3, **caractérisé par le fait qu'**il comprenne entre 10% et 14 % de ladite fibre soluble.
